# EUROPEAN PATENT APPLICATION

(11) **EP 1 146 118 A1**
(43) Date of publication of application: **17.10.2001**
(21) Application number: 00900388.0
(22) Date of filing: 14.01.2000
(51) Int. Cl.: C12N 5/06

(54) **ORGANS FOR TRANSPLANTATION INDUCED IN VITRO**

(30) Priority: 29.01.1999 JP 2107799
(71) Applicant: Japan Science and Technology Corporation, Kawaguchi-shi, Saitama 332-0012 (JP)
(72) Inventor: ASASHIMA, Makoto, Toshima-ku, Tokyo 170-0005 (JP); ARIIZUMI, Takashi, Kawasaki-shi Kanagawa 214-0036 (JP); CHAN, Te-chuan, Tokyo 153-0041 (JP)
(74) Representative: Harding, Charles Thomas
(86) International application number: JP0000148
(87) International publication number: WO0044881

(57) **Abstract**

The object of the present invention is to provide organs for transplantation to open a way to innate kidney diseases or the like of higher organisms by establishing protocol of in vitro induction of organs and in vivo transplantation by means of development engineering or organ engineering to evaluate whether the in vitro induced organ can actually function in vivo. The object of the present invention will be attained by preparing organs for transplantation capable of functioning in vivo when transplanted following culture of in vitro induced kidney, heart, pancreas, lever or enteric canal or the like up to certain stages corresponding to the stages of the recipients of the same species in the presence of TGF- β family such as activin or the like. The stages can be examined by using genome DNA, whose expression corresponds to the stage of the in vitro induced organ, as a molecular marker.

## Description

### Field of the Invention

The present invention relates to development engineering or organ engineering, more particularly in vitro induced organs for transplantation such as kidney, heart, pancreas, liver, enteric canal, notochord, skeletal muscle, leukocyte, erythrocyte, lymphocyte and the like, which can function in vivo when they are in vivo transplanted, and their preparation method and evaluation method.

### Background of the Invention

Every multicellular organism starts its development by fertilization and is completed as an individual having various organs and well-balanced system by undergoing cell division (cleavage) and cell differentiation. The differentiation process is highly complicated and is thought that important interactions between cells called inducing phenomena take place in many steps of differentiation stratum. The clarification of "molecule which dominates morphogenesis" is said to be the most significant. Amphibian embryos are often and mostly used as materials for these studies, nevertheless, the basic rule of body formation is common to all the vertebrates and homologous genes are known to have quite a similar function among different species.

Amphibian embryo has conventionally been regarded as a valuable material in the field of experimental embryology with which many studies have been made. This is because Amphibian egg fertilizes and develops externally, its large egg makes embryo operation possible, and its time course changes can easily be observed. Amphibian dorsal blastopore lip of gastrula is a special region and when it is transplanted into ventral side of other embryo, a secondary embryo including head or body-tail part is induced. This is why dorsal lip of blastopore is named "organizer" as a central region of morphogenesis which determines the embryo system. It is well known that organizer induces central nervous system by functioning to presumptive ectoderm during invagination of the primitive gut, meanwhile the organizer itself differentiates into dorsal mesoderm.

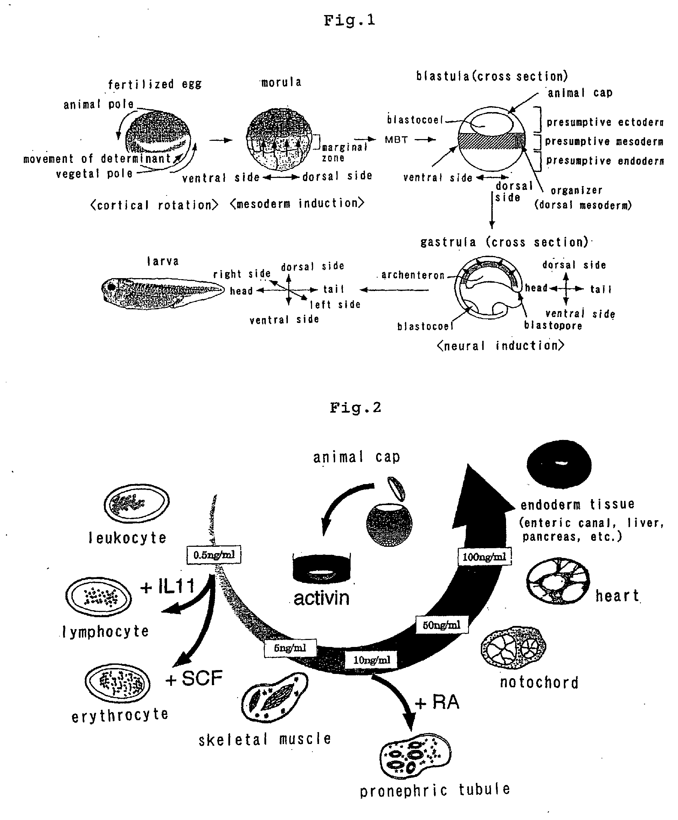

Further, a non-fertilized egg of Xenopus originally contains vertical axis which combines animal pole and vegetal pole, whereas its dorsoventral axis and mediolateral axis are not yet determined. It is known that, when fertilized, rotation occurs between cortex and inner cytoplasm of the egg resulting in that the sperm intruding side becomes the future ventral side and the opposite side becomes the future dorsal side, that sperm always intrude into the animal hemisphere and 180 degree opposite of the intrusion point becomes future blastopore, and that cortical rotation deviates distribution of body axis determinant factor (determinant) included in cytoplasma of fertilized egg, which results in the establishment of dorsoventral axis. A schematic view of the process of Xenopus development is shown in Fig.1.

As is understood from Fig.1, mesoderm induction is the primary induction which starts when cleavage progresses and the embryo reaches at morula stage. The presence of the induction is experimentally proved as a phenomenon in which blastomere of the vegetal hemisphere functions to blastomere of the animal hemisphere to induce mesoderm in the zone. At that time, the vegetal hemisphere blastomere has the inducing gradient along dorsoventral axis, and the ventral blastomere induces ventral mesoderm and the dorsal blastomere induces dorsal mesoderm including organizer. Thus the mesoderm induction is known to localize mesoderm and specify the organizer region. Further, the mesoderm induction mentioned is not specific to amphibians but is involved in the formation of mesoderm and organizer in early embryo of all the vertebrates.

Further, some chemical agents are thought to be transported from cell to cell during the inducing phenomenon. In mesoderm induction, the one secreted from the cell in the vegetal hemisphere to the side of the animal hemisphere is called mesoderm inducing factor. The fixation of mesoderm inducing factor existing in the embryo is one of the most critical theme in embryology. A variety of embryo operations have long been exercised using amphibian embryos and assay systems for inducing factor has already been established. For instance, animal cap assay as a method of direct investigation of mesoderm inducing factor activity, and injection assay as a method of investigating influence on normal development by injecting mRNA of major applicant factor, receptor molecules or the like, into 2-cell to 8-cell stage fertilized eggs are known respectively. The present inventors reported for the first time by the above mentioned animal cap assay (Roux' Arch. Dev. Biol. 198:330-335, 1990), that activin, a cell growth factor of TGF-β family, contains mesoderm inducing activation.

On the other hand, three kidneys, pronephros, mesonephros, and metanephros, are developed in succession in the course of development process of vertebrates, and function as the excretory system. These kidneys are derived from intermediate mesoderm and are formed temporally and spatially well-defined. Especially, the pronephros is the excretory organ established the earliest with quite a simple structure. The basic unit of the kidneys, the nephron, varies in number in three types of kidneys, yet its cytological features are common to them all. Thus, the pronephros is known to provide an attractive model system for studying the mechanism of kidney organogenesis. The present inventors have established a relatively simple experimental system for inducing pronephros (pronephric tubule), and the pronephric tubule has been shown to be induced when Xenopus blastula-stage ectoderm is treated with activin and retinoic acid. The pronephric tubule is shown to express several molecular markers that are expressed in a tadpole pronephros. But whether the pronephros (pronephric tubule) can function as an organ when in vivo transplanted has been unknown.

So far, many attempts have been made worldwide to establish the experimental system to induce kidney in amphibian embryo, or pronephros in vitro, and that inducing only pronephros specifically has been thought to be impossible. As aforementioned, the present inventors have established the experimental system to differentiate pronephros only by treating Xenopus blastula ectoderm with activin and retinoic acid that are bioactive agents. Still, the in vitro induced pronephros will be acknowledged for the first time as an organ for transplantation when it is confirmed to actually function as an excretory organ when in vivo transplanted.

### Disclosure of the Invention

The subject of the present invention is to provide an organ for transplantation to open a way to the transplantation treatment for such as innate kidney diseases or the like in higher organisms by establishing in vitro induction of organs and the method of in vivo transplantation, by taking advantage of development engineering or organ engineering in order to evaluate whether in vitro induced organs actually function in vivo.

The present inventors have demonstrated the following fact for the first time in the world that Xenopus embryo ectoderm (the cell population with pluripotency) treated with activin and retinoic acid induced differentiation of pronephros, the most basic excretory organ established the earliest in ontogeny, and that when ectoderm oriented to differentiate into pronephros was transplanted into embryo which had been removed pronephros primordium (a region where pronephros is formed originally) following the culture of up to a certain stage corresponding to the pronephros primordium stage, the in vitro induced pronephros functioned in vivo. They have confirmed that in vitro induced organ can be transplanted in vivo and the present invention has accomplished.

The present invention relates to a preparation method of organs functioning in vivo when in vitro induced organ is transplanted into a recipient of the same species, wherein the preparation method of in vitro induced organ for transplantation is characterized in that the organ is cultured to a certain stage corresponding to the stage of the recipient. The present invention also relates to: the preparation method of in vitro induced organ for transplantation according to claim 1 characterized in that a certain stage corresponding to the recipient is the same stage as the recipient: the preparation method of in vitro induced organ for transplantation according to any one of claims 1 or 2 characterized in that the certain stage is examined by using genome DNA which expresses corresponding to the stage of the in vitro induced organ as a molecular marker; the preparation method of in vitro induced organ for transplantation according to any one of claims 1 to 3 characterized in that the in vitro induced organ is an organ induced from ectoderm region which has been cut off from the blastula; the preparation method of in vitro induced organ for transplantation according to any one of claims 1 to 4 characterized in that in vitro induction takes place in the presence of TGF (Transforming Growth Factor)-β family. The present invention further relates to the preparation method of in vitro induced organ for transplantation according to claim 5 characterized in that TGF (Transforming Growth Factor)-β family is activin; the preparation method of in vitro induced organ for transplantation according to any one of claims 1 to 4 characterized in that in vitro induction takes place in the presence of activin and retinoic acid; the preparation method of in vitro induced organ for transplantation according to any one of claims 1 to 7 characterized in that the organ is selected from kidney, heart, pancreas, liver, enteric canal, notochord, skeletal muscle, leukocyte, erythrocyte and lymphocyte; the preparation method of in vitro induced organ for transplantation according to any one of claims 1 to 8 characterized in that a living organism of the recipient is embryo; and the preparation method of in vitro induced organ for transplantation according to any one of claims 1 to 9 characterized in that the recipient belongs to vertebrate.

Still further the present invention relates to an in vitro induced organ for transplantation characterized in that the organ is prepared by the preparation method of in vitro induced organ for transplantation according to any one of claims 1 to 10; an evaluation method of in vitro induced organ of non-human animal characterized in that in vitro induced organ of non-human animal is transplanted into a living organism of non-human recipient of the same species; an evaluation method of in vitro induced organ of non-human animal characterized in that the in vitro induced organ of non-human animal is cultured up to a certain stage corresponding to the stage of non-human recipient of the same species, wherein the cultured organ is in vivo transplanted into the recipient. It still further relates to the evaluation method of in vitro induced organ of non-human animal according to claim 13 characterized in that the certain stage is examined by using genome DNA which expresses corresponding to the stage of in vitro induced organ as a molecular marker; the evaluation method of in vitro induced organ of non-human animal according to either one of claims 13 or 14 characterized in that the in vitro induced organ is an organ induced from ectoderm region which has been cut off from the blastula; the evaluation method of in vitro induced organ of non-human animal according to any one of claims 13 to 15 characterized in that in vitro induction takes place in the presence of TGF (Transforming Growth Factor)-β family; and the evaluation method of in vitro induced organ of non-human animal according to any one of claims 13 to 16 characterized in that the organ is selected from kidney, heart, pancreas, liver, enteric canal, notochord, skeletal muscle, leukocyte, erythrocyte and lymphocyte.

### Brief Description of the Drawings

Fig. 1 is a schematic view explaining the process of Xenopus development
Fig.2 is an illustration explaining the organogenesis of animal cap induced by activin which is a mesoderm inducing factor or by other bioactive agents.
Fig.3 is photographs of the pronephric tubules induced by animal cap treated with mixed solution of activin and retinoic acid.
Fig.4 is a schematic view showing transplantation of ectoderm, which is to differentiate into the pronephric tubule, to either one of Xenopus bilateral pronephrectomy sides of embryo.
Fig.5 is a photograph of pronephrectomy Xenopus tadpole larva showing severe edema (above), and normally developed Xenopus tadpole larva having a graft differentiated into the pronephric tubule on its one side (below).
Fig.6 is a table showing survival curve of transplants with grafts induced to pronephric tubules and pronephrectomy embryos without being transplanted at stage 20 of Xenopus.

### Best Mode to Carry out the Invention

The preparation method of in vitro induced organ for transplantation of the present invention is a preparation method of an organ which functions in vivo when in vitro induced organ is transplanted into a living organism of a recipient of the same species, characterized in that the organ is cultured up to a certain stage corresponding to the stage of the recipient.

Organs induced in vitro of the present invention include, for convenience, tissues such as notochord, skeletal muscle, leukocyte, erythrocyte, lymphocyte and the like besides organs that compose internal organs or the like of animals such as kidney, heart, pancreas, liver, enteric canal and the like. Further, the recipients of the present invention include human besides organisms of the same species as in vitro induced organs, namely vertebrate of the same "species" taxonomically, for example, non-human animals like Amphibia such as newts or frogs or the like, Pisces, Aves, or Mammalia such as mouse or the like.

Using ectoderm region (animal cap) removed from the blastula is preferable for in vitro induction of organs. As being non-differentiated cell population, presumptive ectoderm region of the blastula turns into epidermis-like cell lump (formless epidermis) without differentiating the tissues when there is no mesoderm inducing activity agent in the culture solution. But when mesoderm inducing activity exists in the culture solution, it becomes a tissue lump including mesoderm such as hemocyte, muscle, notochord or the like. For examination of the cell differentiation or the stages, it is preferable to conduct quantitative analysis using many kinds of gene markers or antibodies simultaneously with observation of the tissues.

Whether in vitro induced organs can function in vivo when transplanted to a living organism of the recipients of the same species depends largely on the stage at which transplantation is performed. Therefore in vitro induced organs for transplantation need to be cultured up to a certain stage corresponding to the recipients of the same species, that is up to the stage where in vivo transplanted organ can function in the living organism. In many cases, when an organ is transplanted to in vivo of a recipient after being cultured up to the same stage as the recipient, the capability of functioning in vivo when transplanted into a living organism increases. For example, when the organ of the recipient, which is the object for transplantation, is in the stage of primordium, it is preferable to culture the organ for transplantation in physiological saline or the like up to the same primordium stage. It is also preferable, when the object organ for transplantation is in a definitive organ stage, to culture the organ for transplantation in serum or the like up to the stage of the definitive organ. Here a stage means each stage to which the progression process of ontogeny, where one living organism finishes as an adult starting from a fertilized egg or a parthenogenetic egg, is divided into and numbered. In Amphibia, for example, the progression process to metamorphosis is divided into about 60 stages.

Testing stages of the in vitro induced organs can be performed by observing the tissues and by the method using antibodies as above mentioned. But more defined testing can be performed by using genome DNA, which expresses corresponding to the stage of in vitro induced organ, as a molecular marker. For example, in case of normal development of Amphibia embryo, expression of genome DNA rapidly increases from the middle blastula stage where cleavage took place 12 times. After that, it is known that the quantity and variety of genes that express corresponding to embryo stages show changes in strict tiers. In case of in vitro induced organs treated with activin, these tiers are also entirely observed as shown in Table 1. Especially as marker genes relating to kidney differentiation of Xenopus, the followings are exemplified: Xlim-1 expressed at stages 11 to 31, Xpax-2 expressed at and after stage 20, XCIRP or XeWT1 expressed at and after stage 22, Na⁺K⁺-ATPase expressed at and after stage 27, and Cap-1 or X1caax-1 expressed at and after stage 31.

**Table 1.**

| Genes | The starting time of expression | Properties |
|---|---|---|
| Primary response genes | | |
| Mix.1 | about 30 min. | homeobox gene, similar to paired of Drosophila |
| goosecoid | about 30 min. | homeobox gene, similar to gooseberry and bicoid of Drosophila |
| Xlim-1 | about 2 hours | homeobox gene with LIM domain |
| Xbra | 90-250 min. | similar to mouse Brachyury (T) |

| Secondary response genes | | |
|---|---|---|
| XIHbox 1 | about 11 hours | homeobox gene, similar to Drosophila antennapedia |
| XIHbox 6 | about 13 hours | homeobox gene, similar to Drosophila abdominal-B |
| Xwnt-8 | about 16 hours | similar to oncogene Wnt-1 (int-1) |
| XMyoD | about 16 hours | similar to mouse MyoD |
| a-actin | about 19 hours | actin gene of myocardium and skelton |
| N-CAM | about 19 hours | gene of neural cell adhesion molecule |

In vitro organ induction of the present invention can be performed in the presence of TGF- β family. TGF is also called transforming growth factor and functions to transform normal growing property of the cell to transforming growth property, alone or in cooperation with other factors when added to the culture environment. TGF-α having common receptor with epidermal growth factor (EGF) and TGF-β showing TGF activity in cooperation with TGF- α are known as the kinds of TGF. As TGF-β family, TGF-β, activin, inhibin, Mullerian duct inhibiting factor are exemplified. Among these, activin is most preferable from the view point of mesoderm inducing activity.

Mammalia activin, which is secreted from ovary membrane granulocyte and which is a cell growth factor with activity promoting follicle-stimulating hormone secretion (FSH) from anterior hypophysis and which exists as a dimer showing S-S binding to inhibin β chain subunit having FSH secretion inhibiting factor activity, induces mesoderm tissues in presumptive ectoderm with the concentration of 0.1 ng/ml or more. Mammlia activin can cause expression of mesodermal marker genes such as muscle actin or homeobox gene or the like, and has property that it can induce all the mesodermal tissues from ventral side to dorsal side in every assay system. Function of activin is obviously concentration dependent, so that the higher the concentration is, differentiation occurs the more in dorsal mesoderm. Hemocyte, coelomic epithelium and mesenchyme differentiate in the animal cap treated with about 0.3 to 1.0 ng/ml of activin, muscle differentiate at the concentration of 5 to 10 ng/ml, and notochord the most dorsal mesoderm differentiate at the concentration of 50 to 100 ng/ml respectively.

Meanwhile, FGF (Fibroblast Growth Factor) too differentiate hemocyte, mesenchyme and muscle depending on the concentrations, still notochord cannot be induced even at high concentrations (30 to 120 ng/ml), so that in vitro induction of organ is preferable to be performed in the presence of TGF-β family such as activin or the like. Further, neural tissues are often observed inside activin-treated grafts, which is thought to be a result of neural induction to non-differentiated cell by organizer induced by activin of high concentration. These show that activin not only merely induces tissue differentiation but is deeply involved in dorsoventral axis determination. Organogenesis induction by activin to animal cap is shown in Fig.2.

Still further, activin can induce various organs through interaction with inatravital agents of some other kinds (see Fig.2). The present inventors have demonstrated that kidney differentiation is induced by activin/retinoic acid treatment of Xenopus animal cap at an adequate concentration and that hemocyte differentiation can be controlled by activin/BMP (Bone Morphogenesis Protein) or by IL-11, SCF (Stem Cell Factor). Retinoic acid shown as RA in Fig.2 as mentioned above, is also called Vitamin A acid and is an important metabolic product of Vitamin A (retinol) which is indispensable for normal development of animals and for differentiation and growth of epithelium tissues or cartilage. It has morphogen-like function in the process of vertebrate development, which function is demonstrated by controlling gene expression via retinoic acid receptor.

The present invention will be explained below in more detail with the embodiments, but the technical scope of the invention will not be limited to these embodiments.

### Embodiment 1

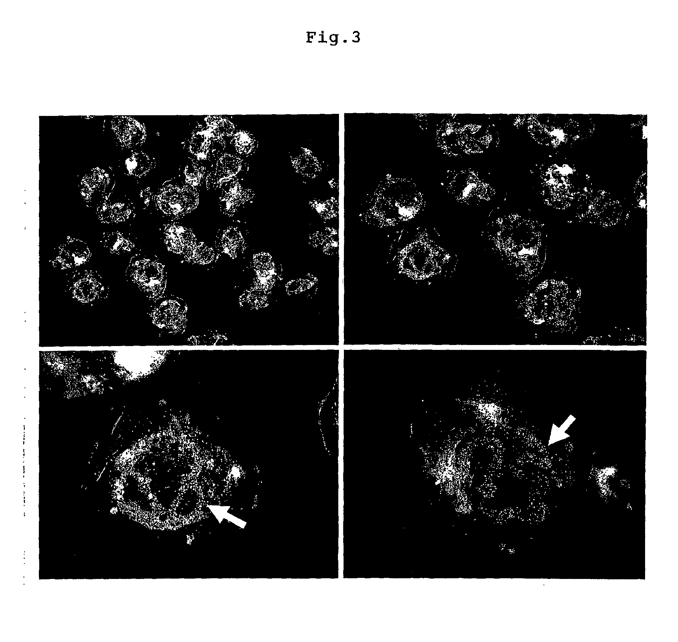

The differential pattern of the in vitro cultures treated with activin and/or retinoic acid was examined. Ectoderm of Xenopus blastula (stage 9) was taken out and was treated with 10ng/ml activin (human recombinant activin A) solution, 10⁻⁴ M retinoic acid (R-2625, Sigma, USA) solution, or mixed solution including 10ng/ml activin and 10⁻⁴ M retinoic acid for 3 hours, which was then cultured for 4 days in physiological saline (Steinberg's solution: SS) to investigate in vitro differential and inducing pattern of the explants. The results are shown in Table 2. The parenthesized numbers in Table 2 are the ratio (%) to all test samples (same as Table 3). As shown in Table 2, differentiation into pronephric tubules at a high frequency was observed after 4 days of culture when treated with mixed solution including 10ng/ml activin and 10⁻⁴ M retinoic acid, and 86% of the treated ectoderms displayed formation of pronephric tubules. A photograph of the formed pronephric tubules (arrow) is shown in Fig.3. Treatment with activin alone resulted in differentiation into muscle, mesenchyme, coelomic epithelium, and neural tissue or the like in ectoderms. No differentiation was observed when treated with retinoic acid alone or without any treatment, and ectoderms were led to be formless lumps of epidermal cells.

### Embodiment 2

The in vivo differential pattern of the activin and/or retinoic acid treated graft was examined by transplantation method. The graft (0.3 mm X 0.3 mm) prepared in a same way as in Embodiment 1 was transplanted into left side of Xenopus embryo (stage 20), region where pronephros primordium had been removed (cut off), then in vivo differential and inducing pattern of the grafts was examined after 5 days of transplantation. The results are shown in Table 2 as Embodiment 1. What is understood from Table 2 is that: ectoderm treated with mixed solution including 10ng/ml activin and 10⁻⁴ M retinoic acid displayed pronephric tubules differentiation at a high frequency at day 4: all the treated ectoderm formed pronephric tubules in the host embryos: in case of treatment with activin alone induced muscle, coelomic epithelium, and epidermis in ectoderm, with very few of grafts differentiated into pronephric tubules: and grafts without any treatment and with retinoic acid treatment alone displayed little differentiation and the ectoderm forms a part of epidermis in the host embryo. Differentiation into pronephric tubules was confirmed by labeling the grafts with fluorescent pigment consists of 1% fluorescein-dextran-amine (FDA; D-1820, Molecular Probes) and by tracing them.

**Table 2**

| | In vitro | | | | In vivo | | | |
|---|---|---|---|---|---|---|---|---|
| Conc.of activin A(ng/ml) | 0 | | 10 | | 0 | | 10 | |
| Conc.of retinoic acid (M) | 0 | 10⁻⁴ | 0 | 10⁻⁴ | 0 | 10⁻⁴ | 0 | 10⁻⁴ |
| The number of test samples | 37 | 32 | 35 | 37 | 14 | 15 | 23 | 23 |
| Formless epidermis | 37 | 32 | 0 | 1 | 14 | 15 | 0 | 0 |
| | (100) | (100) | | (3) | (100) | (100) | | |
| Epidermis | 0 | 0 | 34 | 35 | 0 | 0 | 6 | 8 |
| | | | (97) | (95) | | | (26) | (35) |
| Neural tissue | 0 | 0 | 28 | 2 | 0 | 0 | 1 | 0 |
| | | | (80) | (5) | | | (4) | |
| Muscle | 0 | 0 | 32 | 2 | 0 | 0 | 16 | 0 |
| | | | (91) | (5) | | | (70) | |
| Pronephric tubule | 0 | 0 | 0 | 32 | 0 | 0 | 4 | 23 |
| | | | | (86) | | | (17) | (100) |
| Mesenchyme | 0 | 0 | 27 | 7 | 0 | 0 | 0 | 0 |
| | | | (77) | (19) | | | | |
| Coelomic epithelium | 0 | 0 | 3 | 7 | 0 | 0 | 10 | 8 |
| | | | (86) | (19) | | | (43) | (35) |
| Gut | 0 | 0 | 0 | 0 | 1 | 1 | 8 | 4 |
| | | | | | (7) | (7) | (35) | (17) |

### Embodiment 3

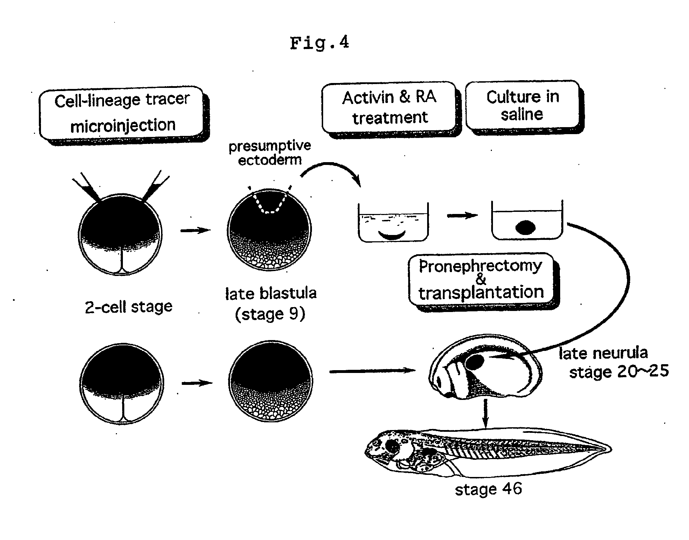

Ectoderm, to be differentiated into pronephric tubule, which was differentiated and induced in vitro by using mixed solution of activin and retinoic acid, and which was then examined as being at stage 20 by being detected with genome DNA, Xpax-2 as a molecule marker, which expresses corresponding to stage 20, was transplanted into one of Xenopus embryo side where bilateral pronephric primordia (located under somites 3 to 5) had been removed at stages 20, 23, and 25. Whether the grafts can function in vivo was investigated. A schema of the transplantation is shown in Fig.4. Bilateral pronephrectomy Xenopus which had not been transplanted was used as control. The results are shown in Table 3.

**Table 3**

| Stage | 20 | | 23 | | 25 | |
|---|---|---|---|---|---|---|
| | Control | Graft | Control | Graft | Control | Graft |
| The number of test samples | 96 | 141 | 36 | 48 | 10 | 19 |
| Normal embryo | 7(7) | 29(21) | 1(3) | 4(8) | 0 | 1(5) |
| Edema embryo | 89(93) | 112(79) | 35(97) | 44(92) | 10(100) | 18(95) |

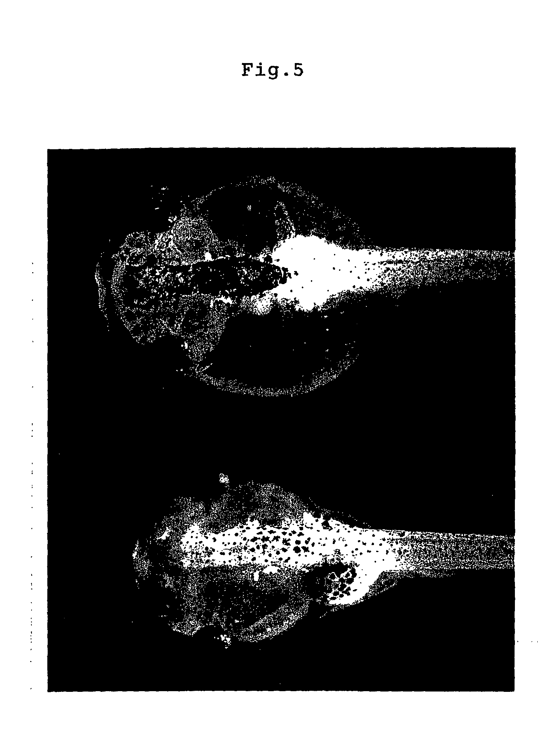

As shown in Table 3, 29 (21%) of the 141 stage 20 transplants, 4 (8%) of the 48 stage 23 transplants, and 1 (5%) of the 19 stage 25 transplants developed normally with no edema-formation, and the transplant pronephros functioned as excretory organ suppressing edema-formation. Meanwhile only 7 (7%) of the 96 stage 20 control embryos that underwent bilateral pronephrectomy, and 1 (3%) of the 36 stage 23 control embryos developed normally without edema-formation. All 10 of the stage 25 control embryos formed edema being unable to excrete water. A photograph of a normally developed tadpole (below) and a tadpole with edema-formation (above) is shown in Fig.5.

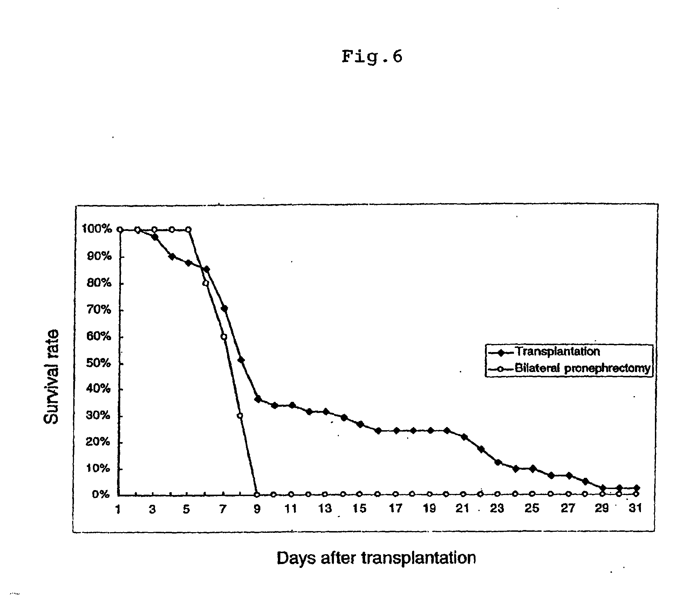

The relationship between post-operation days of the transplantation or pronephrectomy and the survival rate at stage 20 is shown in Fig.6. The following facts are understood from Fig.6. When only pronephrectomy was carried out and not the transplantation, the test samples showed edema-formation and died within 9 days. In contrast, when transplantation was performed, 29 (21%) of the 141 transplants developed normally without edema-formation as above mentioned, with some of them even survived for more than 1 month.

As is known from the experiments using Rana dalmatina of Ranidae, the edema-formation was observed following removal of bilateral pronephric primordia from an embryo at primary stage for being incapable of water excretion. In the transplantation test of the above Embodiment 3, when activin/RA-treated ectoderms of stages 20 to 25 are transplanted into the stages 20 to 25 embryos that are removed of pronephric primordium located under somites 3 to 5, the formation of edema was suppressed. The fact draws the conclusion that the grafts functioned as pronephros, an excretory organ. In comparison with the transplantations performed at stage 20, when transplanted at or after stage 23, extremely a small number developed normally. Thus, the stage at which transplantation is performed is a critical element. It was demonstrated that integration of an organ for transplantation into the host significantly decreased when later stage (stage 25) embryo was used.

### Industrial Applicability

The present invention enables in vitro induction of an organ by a relatively simple experimental system using such as activin alone or activin/RA or the like as mesoderm inducing factor, it also enables to prepare an organ which can function in vivo when transplanted in vivo of the recipient of the same species following culture of the in vitro induced organ up to a certain stage corresponding to the stage of the recipient. Further the present invention enables to evaluate whether the in vitro induced organ is suitable for transplantation by performing in vivo transplantation following culture of the in vitro induced organ up to a certain stage corresponding to the stage of the recipient of the same species.

The present invention makes it possible to confirm whether the in vitro induced organ actually functions in vivo. Thereby not only many significant views to clarify structure of formation of kidney or other such organs are demonstrated but also the present invention contributes to establish a new field in developing engineering or organ engineering that involves in vitro organ formation and its in vivo transplantation. Further, the present invention enables organ transplantation fitting immunologically and it will open a way to treat diseases of higher organisms including human such as kidney diseases or the like.

## Claims

1. A preparation method of organs functioning in vivo when in vitro induced organ is transplanted into a recipient of the same species, wherein the preparation method of in vitro induced organ for transplantation is **characterized in that** the organ is cultured to a certain stage corresponding to the stage of the recipient.

2. The preparation method of in vitro induced organ for transplantation according to claim 1 **characterized in that** a certain stage corresponding to the recipient is the same stage as the recipient.

3. The preparation method of in vitro induced organ for transplantation according to any one of claims 1 or 2 **characterized in that** the certain stage is examined by using genome DNA which expresses corresponding to the stage of the in vitro induced organ as a molecular marker.

4. The preparation method of in vitro induced organ for transplantation according to any one of claims 1 to 3 **characterized in that** the in vitro induced organ is an organ induced from ectoderm region which has been cut off from the blastula.

5. The preparation method of in vitro induced organ for transplantation according to any one of claims 1 to 4 **characterized in that** in vitro induction takes place in the presence of TGF (Transforming Growth Factor)-β family.

6. The preparation method of in vitro induced organ for transplantation according to claim 5 **characterized in that** TGF (Transforming Growth Factor)-β family is activin.

7. The preparation method of in vitro induced organ for transplantation according to any one of claims 1 to 4 **characterized in that** in vitro induction takes place in the presence of activin and retinoic acid.

8. The preparation method of in vitro induced organ for transplantation according to any one of claims 1 to 7 **characterized in that** the organ is selected from kidney, heart, pancreas, liver, enteric canal, notochord, skeletal muscle, leukocyte, erythrocyte and lymphocyte.

9. The preparation method of in vitro induced organ for transplantation according to any one of claims 1 to 8 **characterized in that** a living organism of the recipient is embryo.

10. The preparation method of in vitro induced organ for transplantation according to any one of claims 1 to 9 **characterized in that** the recipient belongs to vertebrate.

11. An in vitro induced organ for transplantation **characterized in that** the organ is prepared by the preparation method of in vitro induced organ for transplantation according to any one of claims 1 to 10.

12. An evaluation method of in vitro induced organ of non-human animal **characterized in that** in vitro induced organ of non-human animal is transplanted into a living organism of non-human recipient of the same species.

13. An evaluation method of in vitro induced organ of non-human animal **characterized in that** the in vitro induced organ of non-human animal is cultured up to a certain stage corresponding to the stage of non-human recipient of the same species, wherein the cultured organ is in vivo transplanted into the recipient.

14. The evaluation method of in vitro induced organ of non-human animal according to claim 13 **characterized in that** the certain stage is examined by using genome DNA which expresses corresponding to the stage of in vitro induced organ as a molecular marker.

15. The evaluation method of in vitro induced organ of non-human animal according to either one of claims 13 or 14 **characterized in that** the in vitro induced organ is an organ induced from ectoderm region which has been cut off from the blastula.

16. The evaluation method of in vitro induced organ of non-human animal according to any one of claims 13 to 15 **characterized in that** in vitro induction takes place in the presence of TGF (Transforming Growth Factor)-β family.

17. The evaluation method of in vitro induced organ of non-human animal according to any one of claims 13 to 16 **characterized in that** the organ is selected from kidney, heart, pancreas, liver, enteric canal, notochord, skeletal muscle, leukocyte, erythrocyte and lymphocyte.
